# EUROPEAN PATENT APPLICATION

(11) **EP 0 848 057 A1**
(43) Date of publication of application: **17.06.1998**
(21) Application number: 97119610.0
(22) Date of filing: 10.11.1997
(51) Int. Cl.: C12M 1/22, C12M 1/20

(54) **Airtight slide for double culture media**

(30) Priority: 12.11.1996 IT UD960217
(71) Applicant: TAPPITAL Srl, I-15100 Alessandria (IT)
(72) Inventor: Crimella, Valerio, 15100 Alessandria (IT)
(74) Representative: Petraz, Gilberto Luigi

(57) **Abstract**

Airtight slide for double culture media which can be used for bacterial tamponades from surfaces for microbiological analyses or for the inoculation of samples to be analysed, the slide including a supporting base (13) defining at least two counter-opposed containing seatings, the upper (11a) and the lower (11b), able to house two separate culture medias (12a, 12b), the supporting base (13) including relative raised edges, the upper (14a) and the lower (14b), to contain the relative media (12a, 12b), each containing seating (11a, 11b) cooperating with its own closing stopper (15a, 15b), at least the lower containing seating (11b) cooperating with at least a reticular element (20b) arranged in an intermediate position on the height of the containing seating (11b) and clamped in a stable position inside the containing seating (11b) with which it is associated.

## Description

This invention concerns an airtight slide for double culture media as set forth in the main claim.

The slide is used both to perform tamponades from surfaces whose microbiological state has to be analysed and also for the inoculation of samples to be analysed in a similar manner to that used with Petri slides, as is known to the state of the art.

The state of the art includes those procedures of bacteriological analyses using culture medias suitable to encourage the growth of specific microcellular organisms of which the presence and concentration has to be monitored.

According to the method using Petri slides and "contact" slides, samples of the substance to be analysed are introduced into distinct culture medias, each of which is suitable to identify one or more microcellular organisms which may be present in the sample.

The slides used in this method substantially consist of thin containers, normally made of plastic, with a supporting base and raised edges to contain the culture media with which an airtight lid is associated.

The lid is normally associated with a sealing film and allows the culture media to be preserved from environmental contamination which would prejudice the efficiency and accuracy of the bacteriological analysis.

In bacteriological analyses of a surface, these "contact" slides are used as tampons to make the outer face of the culture media, advantageously at least slightly rounded, adhere to the surface to be analysed.

Apart from traditional slides with a single containing seating, there have been proposals for slides with a double containing seating made both above and below the supporting base.

This solution gives the separate containment, in a single slide, of two different types of culture medias or one single culture media but used for two distinct tampons or for two distinct cultures of an inoculated sample.

Either of the two seatings, upper and lower, may be further divided into compartments so as to make slides with multiple seatings.

This solution however, although it is particularly advantageous, has the disadvantage that at least the culture media/medias housed in the lower seating tend/tends to become detached when there is little cohesion, or when the slide is subjected to stresses, vibrations or knocks as for example when it is transported.

This involves serious disadvantages which compromise the analyses and render the slide partly unusable.

WO 82/00834 describes a structure with a cylindrical slide to contain culture medias defining two containing volumes for different medias arranged adjacent to each other on opposite sides of the structure.

The two volumes are made to communicate, when a temporary packing is removed, by a perforated slide.

This solution teaches to cultivate micro-organisms in the culture media in a first containing volume.

Then, the packing is removed and the second containing volume is filled with a second culture media so that the antibiotic substances produced by the micro-organisms spread through the culture media and prevent the growth of pathogenic organisms inoculated in the second containing volume.

This solution relates to a specific process and does not include the simultaneous bacterial cultivation of different micro-organisms in different medias, but includes the transfer of substances from one containing volume to the adjacent one through the separating perforated slide.

A further disadvantage of slides known to the art is that condensation forms inside the volume which contains the culture media during the incubation period of the bacteria colonies.

This condensation makes it difficult to read the slide and compromises the efficacious result of the measurement.

The present applicant has designed, tested and embodied this invention in order to overcome this problem and to achieve further advantages.

The invention is set forth and characterised in the main claim, while the dependent claims describe variants of the idea of the main embodiment.

The purpose of the invention is to achieve an airtight slide for double culture media, characterised in that it has a great capacity to hold the media so as to substantially prevent the media from becoming detached even when the slide is in a critical condition of vibration or stress or when the media itself lacks cohesion.

Another purpose of the invention is to achieve a simple and inexpensive slide which will facilitate and make the quantification of the bacterial colonies present in the culture media contained therein quicker and more accurate.

A further purpose of the invention is to facilitate the ventilation of the medias during the incubation periods of the bacteria colonies, so as to prevent the formation of condensation which can prejudice the correct reading of the colonies.

The slide according to the invention substantially consists of a supporting base, with raised edges on both sides, defining on its lower and upper face a double containing seating for the media.

According to the invention, at an intermediate position with respect to the relative containing seatings, there are respective reticular elements associated at least with the lower containing seating, and advantageously with both; the reticular elements are suitable to increase the slide's capacity to hold the culture media inside the relative seating, substantially functioning as a reinforcement for the media.

The reticular elements are substantially equivalent in shape and size to the containing seating of the media.

According to a variant, the reticular elements are anchored laterally on the raised edges of the supporting base.

The reticular elements are anchored in such a way as to render them substantially definitively constrained to the relative containing seating.

According to a variant, they are anchored in such a way as to allow the reticular element to be removed from the containing seating, should it be necessary.

In a first embodiment, the reticular elements are anchored simply by the contact between the reticular element and the relative raised edges of the supporting base.

According to a variant, there are undercuts on the raised edges which cooperate with the relative reticular element by abutment or by jointing.

According to another variant, they are anchored by means of hot or ultrasound welding.

According to another variant, they are anchored by means of nail or screw elements, possibly associated with angular supports.

According to another variant, the reticular element is made in a single piece with the slide, directly at the molding stage.

According to another variant, the reticular element is anchored by means of the appropriate adhesives.

According to another variant, during the molding stage, small pins, hooks or other similar attachment elements are made on the supporting base which cooperate with mating attachment elements, for example, holes or eyelets, on the reticular element; the attachment elements are solidly joined together by means of hot or cold riveting once the reticular element has been inserted.

The housing seatings are filled with culture media by pouring the culture media through the reticular elements which in this way are completely drowned therein.

The reticular elements perform a gripping action on the culture media and determine optimum holding conditions which prevent the culture media from becoming detached from the relative containing seating, either upper or lower, even in a critical condition during transport or during the conservation period of the slide.

Thanks to the stable nature of the media, it is moreover possible, during the tamponade step, to act with great pressure on the surface to be analysed without causing any damage to the tampon itself.

According to a variant, the possible modular arrangement of the mesh of reticular elements makes it possible to simplify the operations of counting the bacterial colonies in the culture media, thus making these operations more accurate and therefore more reliable.

According to a variant of the invention, the slide makes it possible to achieve a desired ventilation of the culture media during the step of bacteriological incubation, so as to prevent the formation of condensation which can make measurement difficult.

This ventilation is advantageously achieved for both the counter-opposed containing seatings made on the slide.

According to a first embodiment, the action of ventilation is achieved by unscrewing the air-tight cover and achieving the formation of a slit between the containing seating and the outer environment which allows air to circulate.

According to another embodiment, the action of ventilation is achieved by removing the cover from its first, closed position, wherein the slide is hermetically sealed, and placing it in at least a second closed position wherein it defines a slit through which air can circulate with respect to the inner containing seating.

The attached figures are given as a non-restrictive example, and show some preferential embodiments of the invention as follows:
- Fig. 1: shows a cross section of an airtight slide for double culture media in a first embodiment of the invention;
- Fig. 2: shows a variant of Fig. 1;
- Fig. 3: shows the reticular element according to the invention;
- Fig. 4: shows a part view of a variant of Fig. 1;
- Fig. 5: shows a part view of a variant of Fig. 3;
- Fig. 6: shows a variant of the invention;
- Fig. 7: shows a variant of Fig. 6.

The slide 10 to contain culture medias according to the invention has two distinct and counter-opposed containing seatings, the upper 11a and the lower 11b, filled with distinct portions of culture media, the first 12a and the second 12b.

The containing seatings 11a, 11b are defined by a supporting base 13, placed so as to separate the two in such a way as to prevent contamination between the two culture medias 12a and 12b, and respectively by the upper raised edge 14a and the lower raised edge 14b of the supporting base 13.

The edges 14a, 14b are advantageously convergent inwards so as to hold and contain the culture medias 12a, 12b better.

Each of the two containing seatings 11a, 11b cooperates with a relative hermetically closing stopper, for example with a screw, or joint, or under pressure, etc., respectively 15a, 15b, which holds it in a condition of insulation and keeps the culture medias 12a, 12b free from external contamination which could distort the results of the analyses.

In this case, the closing stoppers 15a, 15b are jointed and are constrained on fins 16 made on the lateral extensions 17 of the supporting base 13.

The lateral extensions 17 include gripper and manipulator means at the end, which consist of fins 18 made in a single piece with the extensions 17.

According to a variant, the closing stoppers 15a, 15b are anchored to the inner part of the fins 18.

The slide 10 is equipped moreover, in correspondence with each of the two containing seatings 11a, 11b, with a reticular element 20 including modular meshes 19 which acts as a reinforcement for the relative media 12a, 12b, increasing the ability of the containing seatings 11a, 11b to retain the culture media 12a, 12b inside. The reticular element 20 has a shape and extension substantially equivalent to the inner extension of the containing seating 11a, 11b inside which it is inserted.

Each of the reticular elements, upper 20a and lower 20b, is located in a desired intermediate position on the height of the relative raised edge 14a, 14b which defines the relative containing seating 11a, 11b, in a position substantially parallel to the supporting base 13.

Once the culture medias 12a, 12b have been poured into the relative containing seatings 11a, 11b, they are arranged above, below and between the meshes 19 of the reticular elements 20a, 20b, drowning the elements 20a and 20b and attaching themselves thereto.

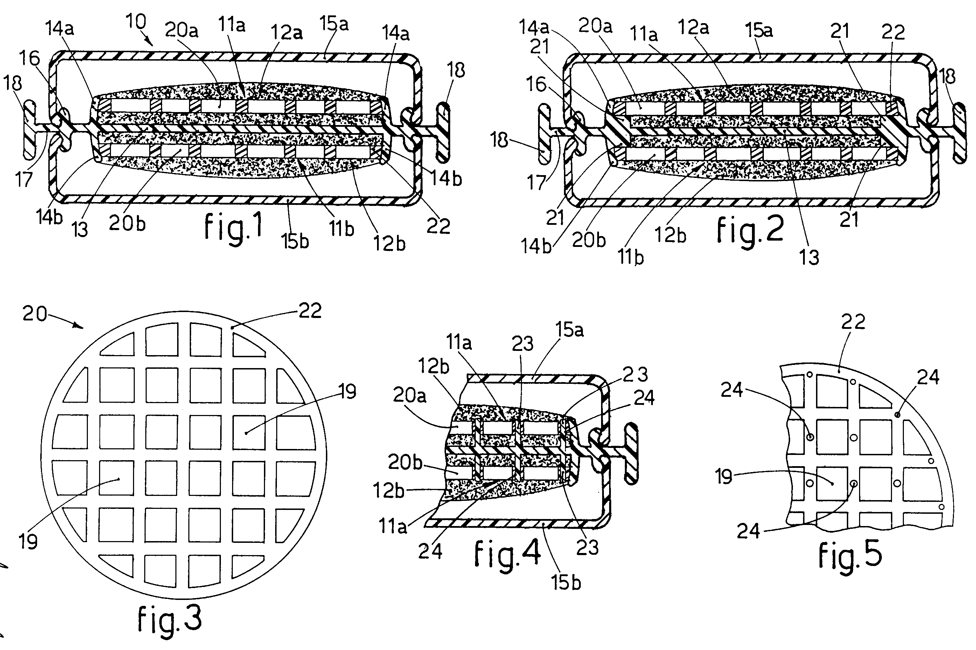

In the case shown in Fig. 1, the reticular elements 20a, 20b are anchored on the edges 14a, 14b of the supporting base 13 by means of simple contact between their own edge 22 and the edges 14a, 14b.

According to a variant shown in Fig. 2, the reticular elements 20a, 20b are anchored due to contact on the relative edges 14a, 14b and are arranged in abutment on undercuts 21 which define the lower positioning limit.

In the embodiment shown partly in Figs. 4 and 5, the supporting base 13 includes attachment elements 23, made in a single piece and in the form of small pins or hooks, which cooperate with mating attachment elements, in this case small holes 24 made on the ribs of the relative reticular element 20.

Once the reticular element 20 has been inserted into the relative containing seating 11a, 11b, the attachment elements 23, 24 are advantageously made indissolubly solid by means of hot or cold riveting.

According to another embodiment, the reticular elements 20a, 20b are attached to the relative raised edges 14a, 14b by means of hot or ultrasound welding. According to another embodiment which is not shown here, the attachment is achieved by using screws or small nails, or by using the appropriate adhesives.

According to another embodiment which is not shown here, the reticular element 20 is made in a single piece with the slide 10, during the molding step.

In the embodiment shown, the reticular element 20a, 20b consists of substantially identical meshes 19; this facilitates and accelerates the counting of the bacterial colonies which can be calculated from the counting of the colonies in the surface unit defined by each individual mesh 19.

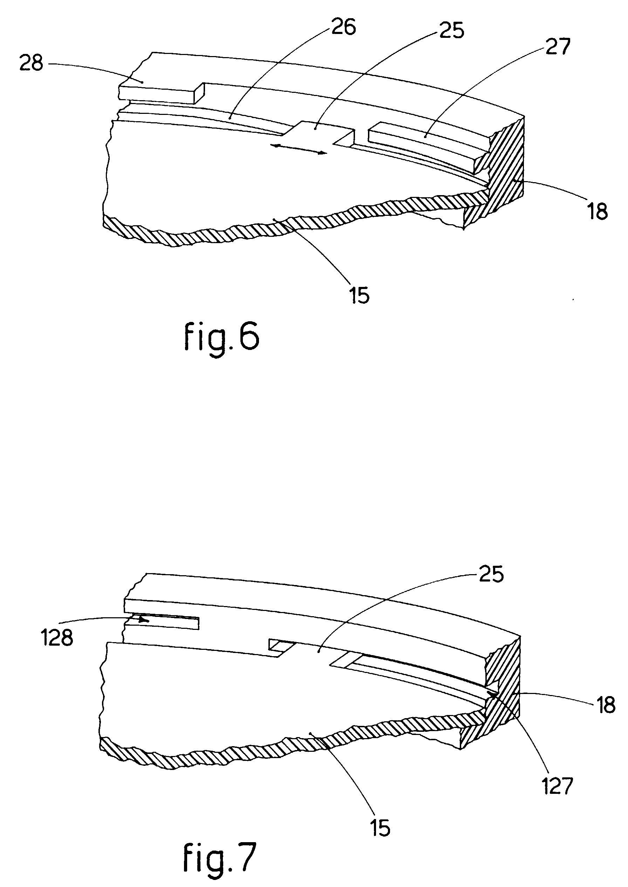

In the variants shown in Figs. 6 and 7, the slide 10 includes means to achieve a desired ventilation of the culture medias 12a, 12b during the incubation period of the bacteria colonies so as to prevent the formation of condensation.

The figures show the case relating to a single containing seating 11, but this solution can be applied equally well also to the counter-opposed containing seating 11.

In the embodiment shown in Fig. 6, ventilation is achieved by means of unscrewing the closing stopper 15.

To be more exact, the closing stopper 15 has lateral fins 25 which cooperate, during the rotations of the stopper 15 as it is screwed/unscrewed, with an inclined sliding plane 26 made on the base 13 of the relative sliding seating 11.

The lateral fins 25 cooperate, in a first position, with a closing fin 27 made on the inner edge of the abutment fin 16, or the peripheral fin 18, according to how the stopper 15 is coupled with the slide 10.

In a second position, by means of partial rotation along the inclined plane 26, the lateral fins 25 cooperate with a ventilation fin 28, located on a higher plane than the closing fin 27.

In this embodiment, the stopper 15 remains constrained to the slide 10 but defines a slit for the passage of air which achieves the desired action of ventilation.

Ventilation may be interrupted at any moment by tightening the stopper 15 and taking it back to a position of closure wherein the lateral fins 25 cooperate with the respective closure fins 27.

According to the variant shown in Fig. 7, on the peripheral edge where the stopper 15 and slide 10 are connected, in the embodiment which includes a jointed closure, there are respective closure grooves 127, arranged on the circumference of a lower plane, and respective ventilation grooves 128, arranged on the circumference of a higher plane.

There may be several levels for the ventilation grooves 128 to achieve more or less intense ventilation.

The closure grooves 127 and ventilation grooves 128 cooperate with the lateral fins 25 in the desired positions according to whether an air-tight closure or a closure with ventilation is desired, and according to the level of ventilation desired.

## Claims

1. Airtight slide for double culture media which can be used for bacterial tamponades from surfaces for microbiological analyses or for the inoculation of samples to be analysed, the slide including a supporting base (13) defining at least two counter-opposed containing seatings, the upper (11a) and the lower (11b), able to house two separate culture medias (12a, 12b), the supporting base (13) including relative raised edges, the upper (14a) and the lower (14b), to contain the relative media (12a, 12b), each containing seating (11a, 11b) cooperating with its own closing stopper (15a, 15b), the slide being characterised in that at least the lower containing seating (11b) cooperates with at least a reticular element (20b) arranged in an intermediate position on the height of the containing seating (11b) and clamped in a stable position inside the containing seating (11b) with which it is associated.

2. Airtight slide as in Claim 1, in which both the upper containing seating (11a) and the lower containing seating (11b) cooperate with relative reticular elements (20a, 20b).

3. Airtight slide as in Claim 1 or 2, in which the reticular element (20) is stably clamped due to contact with the raised edge (14a, 14b) of the relative containing seating (11a, 11b).

4. Airtight slide as in Claim 1 or 2, in which the reticular element (20) cooperates with constraining and/or supporting undercuts (21) made on the inner walls of the raised edges (14a, 14b).

5. Airtight slide as in Claim 1 or 2, in which the reticular element (20) is made in a single piece with the slide (10) during the molding step.

6. Airtight slide as in Claim 1 or 2, in which the reticular element (20) is stably clamped to the raised edge (14a, 14b) of the relative containing seating (11a, 11b) by means of hot or ultrasound welding or by means of glueing.

7. Airtight slide as in Claim 1 or 2, in which the reticular element (20) is stably clamped to the raised edge (14a, 14b) of the relative containing seating (11a, 11b) by means of screw or nail elements.

8. Airtight slide as in Claim 1 or 2, in which the reticular element (20) is stably clamped to the supporting base (13) through the cooperation of mating attachment elements (23, 24) stably associated.

9. Airtight slide as in any claim hereinbefore, in which the reticular element (20) includes modular meshes (19).

10. Airtight slide as in any claim hereinbefore, in which at least one closing stopper (15a, 15b) has a first airtight closed position and at least a second position of ventilation for the relative containing seating (11a, 11b).

11. Airtight slide as in Claim 10, in which the passage of the stopper (15a, 15b) from the first airtight closed position to the second position of ventilation and vice versa is achieved by screwing/unscrewing in cooperation with an inclined sliding plane (26).

12. Airtight slide as in Claim 10, in which the passage of the stopper (15a, 15b) from the first airtight closed position to the second position of ventilation and vice versa is achieved by removing the stopper (15a, 15b) from first closure grooves (127) and repositioning the stopper (15a, 15b) on second ventilation grooves (128) and vice versa.
